# NEW EUROPEAN PATENT SPECIFICATION

(11) **EP 0 331 755 B2**
(45) Date of publication and mention of the opposition decision: **05.02.2003**
(45) Mention of the grant of the patent: 11.08.1993
(21) Application number: 88907808.5
(22) Date of filing: 05.09.1988
(51) Int. Cl.: A61K 9/107

(54) **MEDICINE-CONTAINING FAT EMULSION OF THE TYPE PREPARED IMMEDIATELY BEFORE USE AND PROCESS FOR PREPARING MEDICINE-CONTAINING FAT EMULSION**
ARZNEISTOFF ENTHALTENDE FETTEMULSION DES TYPS "KURZ VOR GEBRAUCH HERGESTELLT" SOWIE VERFAHREN ZUR HERSTELLUNG EINER ARZNEIHALTIGEN FETTEMULSION
EMULSION GRASSE CONTENANT UN MEDICAMENT, DU TYPE PREPARE IMMEDIATEMENT AVANT L'UTILISATION, ET PROCEDE DE PREPARATION D'UNE TELLE EMULSION GRASSE CONTENANT UN MEDICAMENT

(30) Priority: 07.09.1987 JP 22213087; 07.09.1987 JP 22213187
(43) Date of publication of application: 13.09.1989
(73) Proprietor: TEIJIN LIMITED, Osaka-shi, Osaka 541-0054 (JP)
(72) Inventor: TAKAHASHI, Ken, Hino-shi Tokyo 191 (JP); MAKINO, Yuji, Hino-shi Tokyo 191 (JP); SUZUKI, Yoshiki, Hino-shi Tokyo 191 (JP); NARUCHI, Tatsuyuki, Hino-shi Tokyo 191 (JP)
(74) Representative: Hallybone, Huw George
(86) International application number: JP8800889
(87) International publication number: WO89002265

(56) References cited:
- DE-A- 2 441 761
- FR-A- 2 112 759
- FR-A- 2 300 574
- JP-A- 6 174 637
- JP-B- 351 848
- AM. J. HOSP. PHARM., vol. 32, June 1975, pages 582-584, American Society of Hospital Pharmacists, Inc.; C.L. FORTNER et al.: "Fat emulsion vehicle for intravenous administration of an aqueous insoluble drug"
- INTERNATIONAL JOURNAL OF PHARMACEUTICS, vol. 13, 1983, pages 303-312, Elsevier Biomedical Press; A.-A.A. EL-SAYED et al.: "Solubilization and stabilization of an investigational antineoplastic drug (NSC no. 278214) in an intravenous formulation using an emulsion vehicle"
- CHEMICAL ABSTRACTS, vol. 101, no. 20, 12th November 1984, page 375, abstract no. 177301d, Columbus, Ohio, US; L. HUANG et al.: "Coupling of antibodies with liposomes", & LIPOSOME TEHNOL. 1984, 3, 51-62

## Description

### Field of the art

The present invention relates to an extemporaneous preparation type kit of pharmaceutical substance-containing fat emulsion. More particularly, the present invention relates to an extemporaneous preparation type kit, which is composed of (I) a fat emulsion and (II) a pharmaceutical substance composition and capable of giving a pharmaceutical substance-containing fat emulsion having the pharmaceutical substance embedded in the fat emulsion particles by mixing (I) the fat emulsion with (II) a pharmaceutical substance composition containing a pharmaceutical substance and an excipient, and to a method of preparation of the pharmaceutical substance-containing fat emulsion.

### Background of the art

Fat emulsion is a kind of emulsion having very fine particles of fat homogeneously dispersed in water. Especially, an intravenous fat emulsion has been developed for supplying calories to the patients who cannot take them orally and is now commercially available [cf "the fundamentals and preparation of high-calorie transfusion solution": second press; p 74 - 81, (1981); Sigehiko Shimada, YAKUJI NIPPO (Japan)]. Usually, fat emulsion means such intravenous emulsions.

This type of fat emulsion is normally prepared by heating the fat component such as soybean, cotton seed or safflower oil along with an emulsifier such as yolk phospholipid, lecithin or soybean lecithin, and other additives, roughly emulsifying them together with a needed amount of water by means of a homomixer, then finely emulsifying using a jet-type high-pressure homogenizer, and sterilizing with high-pressure steam.

The fat emulsion thus prepared is a homogeneous dispersion of oil-drop particles (of less than 1 micrometer average particle size) coated with an emulsifier on their surface in water. The oil-drop particles coated with an emulsifier on their surface is defined as "fat emulsion particles" here.

In recent years, a variety of trials have been made to develop more reasonable pharmacotherapy with increased efficacy and safety of medicinal substances by controlling the release of the substance from the preparation, preventing the substance from being inactivated in vivo, controlling the distribution of the substance in the living body, or prolonging the retention at the affected part. One of these trials is to develop a satisfactory carrier in which the pharmaceutical substance in the preparation is embedded. In this method, the behavior or efficacy of the pharmaceutical substance largely depend on the absorption, distribution, metabolism, and excretion of the carrier in vivo, differing from a usual preparation in which the pharmaceutical substance is released from the preparation, absorbed, distributed, metabolized and excreted alone in vivo.

The meaning of "a pharmaceutical substance is embedded in a carrier" is defined as follows:

A part or all of the molecules of the pharmaceutical substance constituting the preparation along with the carrier and others such as an excipinet are individually included in the carrier partially or entirely in their molecular bodies.

One of the examples of the entire part of one molecule of the pharmaceutical substance embedded in the carrier is the case in which the pharmaceutical substance is dissolved in the carrier, while an example of one molecule partially included is the case where a part of the molecule exists in the carrier and the rest is exposed to the outside.

Liposome of doubled lipid membrane has been known as such a carrier for a pharmaceutical substance.

Recently, however, trials of utilizing the above-stated fat emulsion particles as a carrier for embedding the pharmaceutical substance have been reported. They describe that the pharmaceutical substance-containing fat emulsion prepared by emulsifying the pharmaceutical substance along with fat and emulsifier prolonged the half-life in vivo of the substance compared with the case where the substance was given without the fat emulsion, and transferred the substance to the target site with selectivity increased.

As examples, are cited a steroid-fat emulsion [Japanese Patent, Laid-open No. 57-16,818 (1982)], a biphenylpropionic acid derivative-fat emulsion [Japanese Patent, Laid-open No. 60-16,923 (1985)] and a prostaglandin E₁-fat emulsion [Japanese Patent, Laid-open No. 58-222,014 (1983)]. The reason why the behavior in vivo of the pharmaceutical substance-containing fat emulsion differs from that of a preparation without the fat emulsion (such as a solution solubilized using a surface active agent) is thought that the pharmaceutical substance is embedded in the fat emulsion particles.

The process for preparation of such a pharmaceutical substance-containing fat emulsion is almost the same as that for preparation of the above-stated fat emulsion. In other words, a pharmaceutical substance to be embedded is dissolved in a specific amount of the oil component, mixed with an emulsifier and other additives, heated, then roughly emulsified along with a needed amount of water using a homomixer, finely emulsified into fine particles of less than 1 micrometer average particle size using a jet-type high-pressure homogenizer, and sterilized with high-pressure steam, after sealed in vessels.

The pharmaceutical substance-containing fat emulsion which is prepared by this method is very useful in medicinal therapy, but has several defects.

The first thing is that the pharmaceutical substance-containing fat emulsion cannot be stored for a long period of time, in case that the pharmaceutical substance is unstable in the fat emulsion. A countermeasure against the defect is, for example, to remove the components which makes the pharmaceutical substance unstable or to add an additive which stabilizes the substance. A former example is a prostaglandin fat emulsion which is stabilized by removing phosphatidyl ethanolamine from the phospholipid [Japanese Patent, Laid-open No. 60-149,524 (1985)]. This method is not, however, the universal one which can stabilize a number of medicines, because the main factors for stabilization are different depending on individual medicinal substances. Therefore, such a pharmaceutical preparation has been desired, as the pharmaceutical substance unstable can be applied in the stabilized form where the substance is embedded in the fat emulsion particles.

The second matter is that there are some pharmaceutical substance-containing fat emulsions which cannot be prepared by the above-stated method. For example, a jet-type high-pressure homogenizer is used in the method, and medicinal substances which explode by a shock cannot be made into the corresponding pharmaceutical substance-containing fat emulsions. Additionally, injection preparations require the high-pressure steam sterilization, but thermally sensible or volatile medicinal substances decompose or dissipate during the steam sterilization. In other words, the medicinal substances which is sensible to heat and highly volatile cannot be made into said pharmaceutical substance-containing fat emulsions.

Accordingly, the development has been desired on the preparations which enable the medicinal substances unstable to heat, shock or the like or highly volatile, to be applied by embedding them into the fat emulsion particles.

The present inventors have made intense investigation on the methods for preparing pharmaceutical substance-containing fat emulsions by mixing a fat emulsion with a pharmaceutical substance composition. Since the fat emulsion is a dispersion of oil or oil particles covered with a surface active agent on their surfaces, the inventors have thought that the pharmaceutical substance is taken into the fat emulsion particles according to the distribution coefficient between water and soybean oil, when the pharmaceutical substance composition is mixed with the oil emulsion.

If a pharmaceutical substance-containing fat emulsion can be prepared by mixing a pharmaceutical substance composition with a fat emulsion, the composition can avoid from the processes of the emulsification by means of a high-pressure jet type homogenizer and/or the sterilization with high-pressure steam. Additionally, if such a kit is provided, as a pharmaceutical substance-containing fat emulsion can be prepared by mixing the pharmaceutical substance composition with the fat emulsion, immediately before the preparation is applied, the stability of the pharmaceutical substance in the fat emulsion offers no longer problems.

FR-A-2300574 concerns a kit of pharmaceutical substances comprising a principal active medicament, water or a physiological serum, vegetable oil and non-immunological protein, in which all these components are presented in separate receptacles.

FR-A-2112759 concerns a method of presenting emulsions (lotions or creams) for cosmetic or dermatological use. To this end one can present separately, but preferably in a common package, an excipient and products which incorporated into the lotion or cream denature themselves.

Chemical Abstracts vol. 101 (1984) No. 20, 177301d mentions a review discussion of covalent coupling of antibodies with fatty acids and incorporation of palmitoyl antibody into liposome bilayer.

Fortner et al. described a method of mixing an anhydrous alcohol solution of an insoluble nitrosourea carcinostatic agent with a fat emulsion to prepare a fat emulsion containing the carcinostatic agent (cf. C.L. Fortner et al., Am. J. Hosp. Pharm. (1975) 32, 582-584).

EI-Sayed et al disclosed a method for preparation of a fat emulsion containing a water-insoluble carbamic acid derivative, a carcinostatic agent, by dissolving the agent in a dimethylacetamide-cremophor® mixture, and mixing the solution with a fat emulsion(cf. A.A. FI-Sayed et al., International Journal of Pharmaceutics, 13, (1983), 303 - 312).

It has been found, however, by the present inventors, that, when an anhydrous alcohol or a dimethylacetamide-cremophor^{R} mixture is mixed with a fat emulsion in order to prepare a fat emulsion containing a sufficiently needed amount of a pharmaceutical substance for treatment, bubbles are formed in the emulsion, and the use of such pharmaceutical substance-containing fat emulsion as an injection is not desirable from a safety point of view.

Consequently, it has been desired that a pharmaceutical substance-containing fat emulsion can be prepared, as the fat emulsion is kept stable, and solvents safe for medicinal substances will be found.

### Disclosure of the invention

The present inventors have made intense studies on pharmaceutical substance compositions which exerts no adverse effect on the stability of the fat emulsion, and attained the present invention by finding following facts:
when a pharmaceutical substance composition containing a pharmaceutical substance as well as saccharides and/or amino acids as an excipient for the substance is mixed with a fat emulsion, the pharmaceutical substance is rapidly transferred and embedded into the fat emulsion particles to give a fat emulsion containing the pharmaceutical substance in a sufficient and necessary amount for treatment, and the excipient can also be added to the fat emulsion without any adverse effect on its stability.

Previously, "the embedment of a pharmaceutical substance into the carrier" was defined, and "a pharmaceutical substance is embedded in fat emulsion particles" has the same meaning as the previous definition except that the carrier is substituted with fat emulsion particles. In more detail, the fat emulsion particles are oil-drop particles which are covered with an emulsifier on their surfaces, and the existence of the whole part of one molecule of the pharmaceutical substance in a fat emulsion particle means that the whole molecule of the pharmaceutical substance dissolves or disperses in only the emulsifier part of the oil particles, in only the oil drop, or in both the emulsifier part and the oil drop.

In the meantime, the partial existence of one molecule of the pharmaceutical substance in the fat emulsion particle shows that a part of one molecule of the pharmaceutical substance distributes in only the oil particle, or in only the emulsifier part or in both the oil particle and the emulsifier part and the rest is outside the fat emulsion particle, in other word, exposed in the water phase.

A pharmaceutical substance which is stable in a conventional preparation process was used to compare a pharmaceutical substance-containing fat emulsion prepared by mixing the above-stated pharmaceutical substance composition with a fat emulsion with another pharmaceutical substance-containing fat emulsion prepared conventionally by dissolving the pharmaceutical substance in the oil and emulsifying the solution by the conventional method and their stability was found to be almost equal.

Thus, when a pharmaceutical substance-containing fat emulsion is prepared by mixing, immediately before the emulsion is applied, a pharmaceutical substance composition with a fat emulsion, a pharmaceutical substance which is unstable in the emulsion and cannot be used in the form of a pharmaceutical substance-containing fat emulsion by the conventional method becomes possible to be used in the form of a fat emulsion. Additionally, the pharmaceutical substance is not subjected to the processes with a jet type high-pressure homogenizer or with a high-pressure steam sterilizer, and pharmaceutical substances which are sensible to heat, shocks or highly volatile also can be prepared into a pharmaceutical substance-containing fat emulsion.

Moreover, the fat emulsion preparations produced by the process according to the present invention can be used as a preparation of excellent drug efficacy with reduced side-effects, as in the conventional fat emulsions, showing a prolonged half life and increased selectivity to the target site.

Thus, the present invention is an extemporaneous type kit of pharmaceutical substance-containing fat emulsion, which is composed of
(1) a fat emulsion including an emulsifier as hereinafter specified, and
(2) a pharmaceutical substance composition containing a pharmaceutical substance as hereinafter specified and a saccharide and/or an amino acid as an excipient, and not at least one solvent selected from liquid polyalkylene glycols, liquid alkylethanol amines and liquid polyhydric alcohols, and
a method for preparation of a pharmaceutical substance-containing fat emulsion by mixing
(1) a fat emulsion including an emulsifier as hereinafter specified with
(2) a pharmaceutical substance composition containing a pharmaceutical substance as hereinafter specified and a saccharide and/or an amino acids as an excipient, and not at least one solvent selected from liquid polyalkylene glycols, liquid alkylethanol amines and liquid polyhydric alcohols, to embed the pharmaceutical substance into particles of the fat emulsion.

### The best embodiment of the invention

The fat emulsion which constitutes the present invention is composed of (a) 0.1 to 50 w/v% of an oil component (b) 1 - 50 parts, preferably 5 - 30 parts of an emulsifier as hereinafter specified, based on 100 parts of the oil component, and (c) a needed amount of water. Further, when needed, an emulsifying auxiliary, a stabilizer, an isotonicity, an antioxidant, a pH regulator also can be added.

The surface of the fat emulsion particles may be modified in order to increase the targeting efficiency toward the target site, strengthen the structure of the fat emulsion particles and inhibit the particles from being taken into the reticuloendothelial system.

The targeting efficiency toward the target site is increased by modifying with, for example, an immunoglobulin which specifically bonds to an antigen or an active fragment in an immunoglobulin or the incorporation into parenchyma cells of liver is increased with a fat emulsion particles having asialofetuin bonded to their surfaces.
The incorporation into the reticuloendothelial system is inhibited by allowing glycofolin, a protein of erythrocyte membrane, to bond to the surface of the the fat emulsion particles.
The structure of the fat emulsion particles is strengthened by modifying their surfaces with pullulan or amylopectin to which palmitic acid is bonded through the primary hydroxyl groups or with a cholesterol which is bonded to their particle surfaces through a crosslinking agent.

As an oil component for the fat emulsion according to the present invention, are cited soybean oil, cotton seed oil, safflower oil, corn oil, sesame oil, olive oil, triglycerides of medium-chain fatty acids, eicosapentanoic acid, and triacetin, but any other oil components also can be used without limitation, as long as they are usable for medicinal purposes. Especially, highly purified soybean oil (which is produced by subjecting normally purified soybean oil to further purification such as steam distillation [cf. H.J. Lips., J. Am. Oil Chemist, Soc., 27, 422 - 423 (1950)], and contains more than 99.9% of triglycerides, diglycerides and monoglycerides) is preferred.

The specified emulsifier for the fat emulsion according to the present invention is a phospholipid, lecithin or hydrogenated lecithin. Any phospholipid, lecithin or hydrogenated lecithin can be used regardless of their origins, and the substances originating from vegetable oil such as soybean oil or from animals such as yolk are employed. Phospholipid is preferably used in its purified form, and the purified product is prepared by a usual fractionation with organic solvents. In other words, for example, crude egg yolk 130 g is dissolved in a cooled n-hexane-acetone 200 ml : 100 ml mixture, then cooled acetone 1,170 ml is gradually added to the solution under stirring. The insoluble fraction is recovered by filtration, then dissolved again in a cooled mixture of 260 ml n-hexane and 130 ml acetone, then cooled acetone 1,170 ml is added under stirring, and the insoluble fraction is recovered by filtration. Finally, the solvent is distilled off, whereby the dried product 60 g is obtained. The product contains 70 - 80 % of phosphatidyl choline and 12 - 25% of phosphatidyl ethanolamine, additionally other phospholipids, phosphatidyl inositol, phosphatidyl serine and sphingomyelin [cf. D.J. Hanahan et al., J. Biol. Chem., 192, 623 - 628 (1950)]. Lecithin is an alias of phosphatidyl choline and obtained by subjecting a purified phospholipid to column chromatography, by chemical synthesis such as esterification of CDP choline with 1,2-diacylglycerol, or by enzymatic reactions such as acylation from Co A into lysolecithin. Many lecithins bear unsaturated fatty acids, and the unsaturated bonds are hydrogenated, for example, by catalytic reduction to give the hydrogenated lecithins resisting to oxidation.

In case of a phospholipid as an emulsifier, an antioxidant is preferably added. Any antioxidant can be used, as long as it is usable as a medicinal component, and vitamin E is particularly suitable.

As an emulsification auxiliary for the fat emulsion according to the present invention, can be used a fatty acid of 6 - 22 carbon atoms, preferably 12 - 20 carbon atoms, or its salt, but any other auxiliary can be employed, as long as it can be added to drugs without limitation. Particularly, naturally occurring fatty acid is suitable. The preferable fatty acids are, for example, stearic acid, oleic acid, linolic acid, palmitic acid or linoleic acid.

The salts of the fatty acids are any physiologically acceptable ones, for example, alkali metal salts such as sodium or potassium salt or alkaline earth metal salts such as calcium salt.

The amount of the emulsification auxiliary added is usually 0.3 (w/v) % based on the fat emulsion.

As a stabilizer for the fat emulsion according to the present invention, are used cholesterol, phosphatidic acid, or polymeric substances, and any other stabilizers can be employed without limitation, as far as they can be added to drugs. Albumin, vinyl polymers and nonionic surface active agents are preferably used as a polymeric substance.

Polyvinyl pyrrolidone or the like are cited as a vinyl polymer.

As a nonionic surface active agent, are used polyalkylene glycol (for example, polyethylene glycol of 1,000 - 10,000, preferably 4,000 - 6,000 average molecular weight), polyoxyalkylene copolymer (for example, polyoxyethylene-polyoxypropylene copolymer of 1,000 - 20,000, preferably 6,000 - 10,000 average molecular weight), hydrogenated castor oil-polyoxyalkylene derivative (for example, hydrogenated castor oil-polyoxyethylene-(40)-ether, -(20)-ether, -(100)-ether), castor oil-polyoxyalkylene derivative (for example, castor oil-polyoxyethylene-(20)-ether, -(40)-ether or -(100)-ether.

The amount of these stabilizers added are different from one another, for example, the cholesterol is less than 0.5 (w/v) %, preferably 0.1 (w/v) %, while the phosphatidic acid is less than 5 (w/v) %, preferably less than 1 (w/v) %.

As the pH controller for the fat emulsion according to the present invention, are used sodium hydroxide, sodium carbonate, sodium hydrogen carbonate or hydrochloric acid, but any other controller can be used, as long as they are usable as a drug.

The isotonicity used for the fat emulsion according to the present invention is, for example, glycerol or glucose.

The pharmaceutical substance composition constituting the present invention is composed of a pharmaceutical substance as hereinafter specified and saccharides and/or amino acids as an excipient. Further, when needed, a stabilizer can be added to increase the stability of the pharmaceutical substance.

The saccharides are, for example, glucose, mannitol, inositol, xylitol or lactose.

The amino acids are, for example, glycine, alanine, arginine.

The stabilizer which can be added to the pharmaceutical substance composition is, for example, a substance to prevent the pharmaceutical substance from being oxidized such as ascorbic acid, EDTA, tocopherol, butyl-hydroxy-toluene, butyl-hydroxy-anisole, propyl gallate, tetra-hydroxyldimethyl, or a preservative such as phenol, sorbic acid, cresol, salicylic acid, propionic acid, methyl p-hydroxybenzoate.

The pharmaceutical substance used in the present invention are specified as the following nine classes of compounds, and the substances of high lipid solubility or oil-soluble derivatives are preferably used:
(1) As steroids, are cited dexamethasone, dexamethasone palmitate, dexamethasone stearate, dexamethasone myristate, hydrocortisone, hydrocortisone palmitate, hydrocortisone stearate, hydrocortisone myristate, prednisoione, prednisolone palimitate, prednisolone stearate, prednisolone myristate or progesterone.
(2) As prostaglandins, are cited prostaglandin A₁, prostaglandin E₁, prostaglandin E₂, prostaglandin F₁ , prostaglandin F₂α, prostaglandin I₂ and their derivatives such as 9(0)-methano-Δ^{6(9α)}-prostaglandin I₁ - (isocarbacylin), 9(0)-methano-Δ^{6(9α)}-prostaglandin I₁ methyl ester, 20-methyl-9(0)-methano-Δ^{6(9α)}-prostaglandin I₁, 6-oxo-prostaglandin E₁, 15-methyl-prostaglandin E₂, 7-thiaprostaglandin E₁ methyl ester, 17,20-dimethyl-7-thiaprostaglandin E₁ methyl ester, 18,18,19,19-tetradehydro-16-methyl-9(0)-metha no-Δ^{6(9α)}-prostagladin I₁, 18,18,19,19-tetrahydro-16,20-dimethyl-9(0)-methano-Δ^{6(9α)}-prostaglandin I₁.
(3) As fat-soluble vitamins, are cited vitamin A₁, vitamin A₂, vitamin A₃, vitamin A₁ palmitate, vitamin D₁, vitamin D₂, vitamin D₃, vitamin D₄, α-tocopherol, β-tocopherol, γ-tocopherol, δ-tocopherol, vitamin K₁, vitamin K₂, vitamin K₃, vitamin K₄, vitamin K₅, vitamin K₆ and their derivatives.
   Particularly, as derivatives of vitamin D₃, are cited activated vitamin D₃, for example, active form of vitamin D₃ bearing a hydroxyl group in the 1α-position such as 1α-hydroxycholecaliciferol (1α-OH-D₃), 1α,25-dihydroxy-chole-calciferol (1α,25-(OH)₂D₃), 1α,24-dihydroxy-chole-calciferol (1α,24-(OH)₂D₃), 1α,24,25-trihydroxy-chole-calciferol (1α,24,25-(OH)₃D₃), 1α-hydroxy-24-oxo-chole-calciferol, 1α,25-dihydroxy-24-oxo-cholecalciferol, 1α,25-dihydroxy-cholecalciferol-26,25-lactone, 1α,25-dihydroxy-cholecalciferol-26,23-peroxylactone, 26,26,26,27,27,27-hexafluoro-1α,25-dihydroxy-cholecalciferol, or active form of vitamin D₃ which does not bear a hydroxyl group in the 1α-position such as 25-hydroxycholecalciferol (25-OH-D₃), 24-oxocholecalciferol, 24,25-dihydroxycholecalciferol (24,25-(OH)₂D₃), 25-hydroxy-24-oxo-cholecalciferol, 25-hydroxycholecalciferol-26,23-lactone, or 25-hydroxychole-calciferol-26,23-peroxylactone.
(4) As an anti-inflammatory, are cited, for example, ibuprofen, flufenamic acid, ketoprofen, indomethacin, and their derivatives.
(5) As a carcinostatic agent, are cited hexamethylmelamine, daunorubicin, doxorubicin, daunomycin, futraful, 5-FU, bleomycin, methotrexate, actinomycin D, mitomycin C, chlorambucil and their derivatives.
(6) As a drug for arrhythmia, are cited propranolol, ajimaline, alprenolol and their derivatives.
(7) As a cardiotonic, are cited digoxin, deslanoside, G-strophanthin, isopreterenol, etilefrine, dopamine, ubidecarenone, and their derivatives.
(8) As a vasodilator, are cited oxyfedrine, carbocromen, dipyridamole, isosorbide nitrate, nitroglycerin and their derivatives.
(9) As a Ca antagonist, are cited nifedipine, nicardipine and their derivatives.

Particularly, prostaglandins (2), their oil-soluble derivatives, vitamin D₃ and its activated derivatives, nitroglycerin are suitably used.

The fat emulsion constituting the present invention is prepared by, for example, the following process:

An emulsifier, for example, phospholipid, and, when needed, aforementioned additives, for example, an emulsification auxiliary, a stabilizer, an antioxidant, and a pH controller are mixed with an oil component such as soybean oil, and they are dissolved by heating at 40 - 75°C to form a solution. The solution is combined with a needed amount of water and emulsified using a usual mixer such as a homomixer at 20 - 80°C to form a rough emulsion. The stabilizer and the isotonicity may be added at this stage.

Then, the rough emulsion is finely particulated at 20 - 80°C by means of a homogenizer (for example, a high-pressure jet type homogenizer such as Manthon-Goulin type homogenizer or ultrasonic wave type homogenizer) to form a homogenized fat emulsion of extremely fine particles. The average particle size of the emulsion is less than 1.0 micron and its storage stability is very good.

In case of a Manthon-Goulin homogenizer, the rough emulsion is homogenized by exposing it to the first stage of pressure ranging from 100 to 150 kg/cm² zero to two times, then the second stage of pressure from 400 to 700 kg/cm² 5 to 15 times.

The fat emulsion is sealed in a vessel and desirably sterilized with high-pressure steam.

As for the preparation of a fat emulsion including surface-modified oil particles, for example, a fat emulsion having an active fragment of an immunoglobulin bonded thereto is prepared as follows:

The active fragment of an immunoglobulin is linked to the fat emulsion particles by introducing a lipophilic substance into the fragment of the immunoglobulin and allowing the fragment to come into contact with a pharmaceutical substance-containing fat emulsion (called method 1 hereinafter) or by adding an lipophilic substance on the preparation of the fat emulsion, and bonding the fragment directly or through a crosslinking agent, after the emulsion is formed. In method 1, the lipophilic substance is suitably introduced into the fragment of immunoglobulin using phospholipid, glycolipid, or fatty acid as a substance. When phosphatidyl ethanolamine (PE) is used as a phospholipid, the fragment of immunoglobulin is allowed to react with N-4-(p-maleimide-phenol)butylphosphatidylethanolamine (p-MPB) resultant from the reaction of the lipid with succinimidyl-4-(p-maleimidephenol) butyrate (SMPB) [cf. J. Biol. Chem. 257, 286 (1982)]. In case of ganglioside, a kind of glycolipid, the lipid is oxidized with periodic acid, then allowed to react with the fragment [cf. Biochem. Biophys. Acta 640, 66 (1981)]. Further, when palmitic acid is used as a fatty acid, palmitic acid ester of N-hydroxysuccin-imide is allowed to react with the fragment of an immunoglobulin [Biochem. Biophys. Acta, 689, 31, (1982)]. The complex between the immunoglobulin fragment and the lipophilic substance is bonded to the surface of the fat emulsion particles by allowing the aqueous solution or suspension of the complex to come into contact with the fat emulsion which has been prepared as stated above. This contact is carried out, for example, by treating 10 parts by weight of the fat emulsion with 0.01 - 10 parts by weight of the complex in the form of 0.1 - 10 % aqueous solution or suspension. The contact temperature is usually 4 - 37°C, the contact time is usually 30 minutes - 24 hours, stirring or shaking is preferred. In method 2, for example, the PE-MPB is added, when the fat emulsion is emulsified, so that the complex becomes 0.01 - 10 w/v%, to effect the contact between the emulsion and the immunoglobulin fragment, thereby the fat emulsion having an immunoglobulin fragment bonded thereto is obtained. The contact is carried out, for example, by treating 10 parts by weight of the fat emulsion with 0.01 - 10 parts by weight of the fragment in the form of 0.1 - 10 % by weight aqueous solution usually at 4 - 37°C for 30 minutes to 24 hours, preferably under stirring or shaking.

The pharmaceutical substance composition containing a pharmaceutical substance and an excipient is preferred to have fine particle sizes and large surface areas, because it must be rapidly admixed to the fat emulsion. To be concrete, the composition is prepared by dissolving the components in distilled water for injection, sterilizing by filtration through a 0.2 micrometer membrane, pouring the filtrate into vessels and freeze-drying.

The amount of the pharmaceutical substance composition containing a pharmaceutical substance and an excipient varies with the quantity of the pharmaceutical substance needed, and is preferably less than 10 parts by weight per 100 parts by weight of the fat emulsion.

The extemporaneous kit of a pharmaceutical substance-containing fat emulsion is applied, for example, as follows:

When applied, the fat emulsion in an ampoule is poured into another ampoule containing the pharmaceutical substance composition and they are thoroughly mixed. The pouring operation can be sterilely conducted. The mixing here is usually achieved by shaking strongly by hand for at least 1 minute or by means of Voltex type shaker for at least 30 seconds at the maximum shaking intensity.

The resultant pharmaceutical substance-containing fat emulsion is preferably applied within a period of time, while the pharmaceutical substance is kept stable.

Further, the pharmaceutical substance-containing fat emulsion which has been prepared from the kit according to the present invention is used as an injection, desirably intravenous injection, but may be also given, after it is mixed with a transfusion solution such as isotonic sodium chloride solution or 5 % glucose solution.

The present invention will be illustrated in more detail by the following examples.

### Example 1

### (1) Preparation of an extemporaneous kit

### a. Fat emulsion

Vials containing 1 ml of a fat emulsion of the following formulation were prepared and the vials were tightly stoppered with rubber caps.

| <Formulation of the fat emulsion> | |
|---|---|
| Purified soybean oil | 50.0 g |
| Purified yolk lecithin | 6.0 g |
| Glycerin for injection | 12.5 g |
| Distilled water for injection | an appropriate amount |
| Total | 500 ml |

### b. A powdery 1α,25-(OH)₂D₃ composition

A powdery pharmaceutical composition containing 1α,25-(OH)₂D₃ was enclosed in vials, after it is prepared as follows:

1α,25-(OH)₂D₃ 0.1 mg was dissolved in Japanese Pharmacopoeia ethanol 0.1 ml. The solution was added to a solution of 50 g of glycine in 1,000 ml of distilled water for injection, stirred, filtered with a membrane filter. The filtrate was poured into vials for freeze-drying 1 ml each. These vials were freeze-dried, replaced the air inside with a nitrogen gas, and tightly closed with sterilized rubber stoppers and aluminum caps respectively. The resultant 1α,25-(OH)₂D₃-containing composition was found to include 0.1 µg of 1α,25-(OH)₂D₃ and 50 mg of glycine, respectively.

### (2) Preparation of 1α,25-(OH)₂D₃-containing fat emulsion

The fat emulsion was taken out a vial(a), and poured into the vial including the 1α,25-(OH)₂D₃ composition (b), and they are thoroughly mixed for 2 - 3 minutes. The ratio of the drug in the liquid phase to the total amount of the drug in the 1α,25-(OH)₂D₃ was almost the same as in the 1α,25-(OH)₂D₃ fat emulsion prepared according to the conventional method. The 1α,25-(OH)₂D₃ was found to be stable at least for 6 hours in the fat emulsion.

### (3) The stability of 1α,25-(OH)₂D₃ in the extemporaneous kit of 1α,25-(OH)₂D₃-containing fat emulsion

After stored at 10°C for 18 months, the extemporaneous kit of 1α,25-(OH)₂D₃-containing fat emulsion prepared in (1) had almost the same content of the drug as in before storage.

In the meantime, the 1α,25-(OH)₂D₃-containing fat emulsion was stored at 10 °C for 18 months, and the content of 1α,25-(OH)₂D₃ was found to be 78% content compared with before storage.

Thus, the extemporaneous kit enables the preparation of 1α,25-(OH)₂D₃-containing fat emulsion free from reduction in the content of 1α,25-(OH)₂D₃, even after storage for a long period of time.

### Example 2

### (1) Preparation of extemporaneous kit

### a. Fat emulsion

Vials containing fat emulsion 1 ml each were obtained in the same manner as in Example 1.

### b. Powdery 1α,24-(OH)₂D₃ composition

A powdery pharmaceutical composition containing 1α,24-(OH)₂D₃ was enclosed in vials, after it was prepared as follows:

1α,24-(OH)₂D₃, 1 mg was dissolved in Japanese Pharmacopoeia ethanol, 1 ml. The solution was added to a solution of 1 g of EDTA, 5g of sodium ascorbate, and 50 g of mannitol in 1,000 ml of distilled water for injection, stirred, filtered with a membrane filter, and the filtrate is poured into vials for freeze-drying 1 ml each. These vials were subjected to freeze-drying, replaced the air inside with nitrogen gas, and tightly closed with sterilized rubber stoppers and aluminum caps respcetively. The resultant 1α,24-(OH)₂D₃-containing composition was found to contain, every vial, 1 µg of 1α,24-(OH)₂D₃, 1 mg of EDTA, 5 mg of sodium ascorbate and 50 mg of mannitol.

### (2) Preparation of 1α,24-(OH)₂D₃-containing fat emulsion

The kit prepared in (1) was used, the fat emulsion was taken out from the vial (a) with an injection syringe and poured into the vial (b) containing the 1α,24-(OH)₂D₃ composition, then they were thoroughly mixed for about 2 minutes to prepare a 1α,24-(OH)₂D₃-containing fat emulsion which contains 1 µg of the drug and is used as an injection.

Meanwhile, for comparison with the extemporaneous kit, 1α,24-(OH)₂D₃, 10 µg was dissolved in soybean oil 1g and converted into a 1α,24-(OH)₂D₃-containing fat emulsion with the formulation in Example 1, (1) a according to the conventional method. The fat emulsion contained 1 µg of 1α,24-(OH)₂D₃ per 1 ml.

Subsequently, in order to enable the fat emulsion which was prepared by the conventional method to be applied as an injection, the emulsion was poured in vials, 2 ml each, the vials were tightly stoppered with rubber caps, and sterilized with high-pressure steam at 121C for 20 minutes. Thus, the content of 1α,24-(OH)₂D₃ reduced to 81 % based on before sterilization.

Thus, the extemporaneous kit has facilitated the preparation of the 1α,24-(OH)₂D₃-containing fat emulsion which can be used as an injection without reduction in 1α,24-(OH)₂D₃ content at the sterilization step in the conventional method.

### The possibility of industrial application

The present invention enables a pharmaceutical substance which has been desired to be given in the fat emulsion form in which it is embedded into the fat emulsion particles, but cannot be stored for a long period of time by conventional techniques, because they were unstable in the fat emulsion, to be administered in the fat emulsion form. Further, the present invention permits a pharmaceutical substance which cannot conventionally be embedded in the fat emulsion, because it is unstable to heat shock or the like or volatile, to be administered in the fat emulsion form in which the substance is embedded in the emulsion fat particles.

Accordingly, the development of pharmaceutical substances-containing fat emulsions which are very useful from a medicinal point of view, because they have very excellent drug efficacy with reduced side-effects has becomes possible and it is very significant.

## Claims

1. An extemporaneous kit of pharmaceutical substance-containing fat emulsion which comprises
(1) a fat emulsion, wherein the fat emulsion is composed of an oil component and an emulsifier, which is at least one selected from phosopholipids, lecithin, and hydrogenated lecithin, and
(2) a pharmaceutical substance composition containing a pharmaceutical substance wherein the pharmaceutical substance is at least one selected from steroids, carcinostatics, prostaglandins. fat-soluble vitamins, anti-inflammatories, cardiotonics, antiarrhythmics, vasodilators, and calcium antagonists and
a saccharide and/or an amino acid as an excipient, and not at least one solvent selected from liquid polyalkylene glycols, liquid alkylethanolamines and liquid polyhydric alcohols.

2. An extemporaneous kit of pharmaceutical substance-containing fat emulsion according to claim 1, wherein the fat emulsion comprises
(a) 0.1 - 50 w/v% of the oil component,
(b) 1 - 50 parts by weight, per 100 parts by weight of the oil component, of an emulsifier, and
(c) an appropriate amount of water.

3. An extemporaneous kit of pharmaceutical substance-containing fat emulsion according to claim 2, wherein the oil component is at least one selected from soybean oil, cotton seed oil, safflower oil, corn oil, sesame oil, olive oil, medium chain fatty acid triglycerides, eicosapentanoic acid and triacetin.

4. An extemporaneous kit of pharmaceutical substance-containing fat emulsion according to any one of claims 1 to 3, wherein the fat emulsion includes fat particles of 1 µm or less particle size on the average.

5. An extemporaneous kit of pharmaceutical substance-containing fat emulsion according to any one of claims 1 to 4, wherein the fat emulsion consists of fat emulsion particles, the surfaces of which are modified.

6. An extemporaneous kit of pharmaceutical substance-containing fat emulsion according to any one of claims 1 to 5, wherein the amount of the pharmaceutical substance composition is less than 10 parts by weight or volume, per 100 parts by weight or volume of the fat emulsion.

7. A method for preparation of pharmaceutical substance-containing fat emulsion which is **characterized by** mixing
(1) a fat emulsion wherein the fat emulsion is composed of an oil component and an emulsifier, which is at least one selected from phosopholipids, lecithin, and hydrogenated lecithin, and
(2) a pharmaceutical substance composition containing a pharmaceutical substance wherein the pharmaceutical substance is at least one selected from steroids, carcinostatics, prostaglandins, fat-soluble vitamins, antiinflammatories, cardiotonics, antiarrhythmics, vasodilators, and calcium antagonists and a saccharide and/or amino acid as an excipient and not at least one solvent selected from liquid polyalkylene glycols, liquid alkylethanolamines and liquid polyhydric alcohols, to embed the pharmaceutical substance into particles of the fat emulsion.

## Patentansprüche

1. Unvorbereitetes Besteck einer Arzneistoff-enthaltenden Fettemulsion, umfassend:
(1) eine Fettemulsion, wobei die Fettemulsion aus einer Ölkomponente und einem Emulgator zusammengesetzt ist, der mindestens einer, ausgewählt aus Phospholipiden, Lecithin und hydriertem Lecithin, ist, und
(2) eine Arzneistoffzusammensetzung, die einen Arzneistoff enthält, wobei der Arzneistoff mindestens einer, ausgewählt aus Steroiden, krebshemmenden Mitteln, Prostaglandinen, fettlöslichen Vitaminen, entzündungshemmenden Mitteln, Cardiotonika, Antiarrhythmika, Vasodilatoren und Calciumantagonisten, ist, und
ein Saccharid und/oder eine Aminosäure als Arzneistoffträger und nicht mindestens ein Lösungsmittel, ausgewählt aus flüssigen Polyalkylenglykolen, flüssigen Alkylethanolaminen und flüssigen mehrwertigen Alkoholen.

2. Unvorbereitetes Besteck einer Arzneistoff-enthaltenden Fettemulsion nach Anspruch 1, wobei die Fettemulsion:
(a) 0,1 - 50 Gew./Vol.-% der Ölkomponente,
(b) 1 - 50 Gew.-Teile, pro 100 Gew.-Teile der Ölkomponente, eines Emulgators und
(c) eine geeignete Wassermenge umfaßt.

3. Unvorbereitetes Besteck einer Arzneistoff-enthaltenden Fettemulsion nach Anspruch 2, wobei die Ölkomponente mindestens eine, ausgewählt aus Sojaöl, Baumwollsamenöl, Distelöl, Maiskeimöl, Sesamöl, Olivenöl, Fettsäuretriglyceriden mit mittlerer Kettenlänge, Eicosavaleriansäure und Triacetin, ist.

4. Unvorbereitetes Besteck einer Arzneistoff-enthaltenden Fettemulsion nach einem der Ansprüche 1 bis 3, wobei die Fettemulsion Fetteilchen von 1 µm oder einer kleineren Teilchengröße im Mittel umfaßt.

5. Unvorbereitetes Besteck einer Arzneistoff-enthaltenden Fettemulsion nach einem der Ansprüche 1 bis 4, wobei die Fettemulsion aus Fettemulsionsteilchen, deren Oberflächen modifiziert sind, besteht.

6. Unvorbereitetes Besteck einer Arzneistoff-enthaltenden Fettemulsion nach einem der Ansprüche 1 bis 5, wobei der Anteil der Arzneistoffzusammensetzung weniger als 10 Gew.-Teile oder Volumenteile, pro 100 Gew.-Teile oder Volumenteile der Fettemulsion, beträgt.

7. Verfahren zur Herstellung einer Arzneistoff-enthaltenden Fettemulsion, **gekennzeichnet durch** Mischen:
(1) einer Fettemulsion, wobei die Fettemulsion aus einer Ölkomponente und einem Emulgator zusammengesetzt ist, der mindestens einer, ausgewählt aus Phospholipiden, Lecithin und hydriertem Lecithin, ist, und
(2) einer Arzneistoffzusammensetzung, die einen Arzneistoff enthält, wobei der Arzneistoff mindestens einer, ausgewählt aus Steroiden, krebshemmenden Mitteln, Prostaglandinen, fettlöslichen Vitaminen, entzündungshemmenden Mitteln, Cardiotonika, Antiarrhythmika, Vasodilatoren und Calciumantagonisten, ist, und
eines Saccharids und/oder einer Aminosäure als Arzneistoffträger und nicht mindestens eines Lösungsmittels, ausgewählt aus flüssigen Polyalkylenglykolen, flüssigen Alkylethanolaminen und flüssigen mehrwertigen Alkoholen, wodurch der Arzneistoff in Fettemulsionsteilchen eingebettet wird.

## Revendications

1. Kit extemporané d'émulsion grasse contenant une substance pharmaceutique, qui comprend
(1) une émulsion grasse, dans laquelle l'émulsion grasse se compose d'un constituant huile et d'un émulsifiant, qui est au moins un émulsifiant choisi parmi les phospholipides, la lécithine et la lécithine hydrogénée, et
(2) une composition de substance pharmaceutique contenant une substance pharmaceutique, dans laquelle la substance pharmaceutique est au moins une substance pharmaceutique choisie parmi les stéroïdes, les cancérostatiques, les prostaglandines, les vitamines liposolubles, les anti-inflammatoires, les cardiotoniques, les anti-arythmiques, les vasodilatateurs et les antagonistes du calcium, et
un saccharide et/ou un acide aminé, comme excipient, et non, au moins, un solvant choisi parmi les polyalkylèneglycols liquides, les alkyléthanolamines liquides et les polyols liquides.

2. Kit extemporané d'émulsion grasse contenant une substance pharmaceutique selon la revendication 1, dans lequel l'émulsion grasse comprend
(a) 0,1 - 50% en poids/volume du constituant huile,
(b) 1 - 50 parties en poids, pour 100 parties en poids du constituant huile, d'un émulsifiant, et
(c) une quantité d'eau appropriée.

3. Kit extemporané d'émulsion grasse contenant une substance pharmaceutique selon la revendication 2, dans lequel le constituant huile est au moins une huile choisie parmi l'huile de soja, l'huile de graine de coton, l'huile de carthame, l'huile de maïs, l'huile de sésame, l'huile d'olive, les triglycérides d'acides gras à chaîne moyenne, l'acide éicosapentanoïque et la triacétine.

4. Kit extemporané d'émulsion grasse contenant une substance pharmaceutique selon l'une quelconque des revendications 1 à 3, dans lequel l'émulsion grasse contient des particules de graisse de granulométrie moyenne inférieure ou égale à 1 µm.

5. Kit extemporané d'émulsion grasse contenant une substance pharmaceutique selon l'une quelconque des revendications 1 à 4, dans lequel l'émulsion grasse est constituée de particules d'émulsion de graisse dont les surfaces sont modifiées.

6. Kit extemporané d'émulsion grasse contenant une substance pharmaceutique selon l'une quelconque des revendications 1 à 5, dans lequel la quantité de composition de substance pharmaceutique est inférieure à 10 parties en poids ou en volume, pour 100 parties en poids ou en volume d'émulsion grasse.

7. Procédé de préparation d'une émulsion grasse contenant une substance pharmaceutique, qui est **caractérisée, en ce que** l'on mélange
(1) une émulsion grasse dans laquelle l'émulsion grasse se compose d'un constituant huile et d'un émulsifiant, qui est au moins un émulsifiant choisi parmi les phospholipides, la lécithine et la lécithine hydrogénée, et
(2) une composition de substance pharmaceutique contenant une substance pharmaceutique, dans laquelle la substance pharmaceutique est au moins une substance pharmaceutique choisie parmi les stéroïdes, les cancérostatiques, les prostaglandines, les vitamines liposolubles, les anti-inflammatoires, les cardiotoniques, les anti-arythmiques, les vasodilatateurs et les antagonistes du calcium, et
un saccharide et/ou un acide aminé, comme excipient, et non, au moins, un solvant choisi parmi les polyalkylèneglycols liquides, les alkyléthanolamines liquides et les polyols liquides, pour effectuer l'inclusion de la substance pharmaceutique dans les particules de l'émulsion grasse.
